# EUROPÄISCHE PATENTSCHRIFT

(11) **EP 3 352 710 B1**
(45) Veröffentlichungstag und Bekanntmachung des Hinweises auf die Patenterteilung: **07.08.2019**
(21) Anmeldenummer: 16770479.0
(22) Anmeldetag: 20.09.2016
(51) Int. Cl.: A61F 2/50, A61F 2/58, A61F 2/68, A61F 2/70, A61F 2/78

(54) **PROTHESENEINRICHTUNG**
PROSTHESIS ASSEMBLY
ENSEMBLE PROTHÉTIQUE

(30) Priorität: 24.09.2015 DE 102015116133
(43) Veröffentlichungstag der Anmeldung: 01.08.2018
(73) Patentinhaber: Otto Bock Healthcare Products GmbH, 1070 Wien (AT)
(72) Erfinder: KALMAR, Janos, 1140 Wien (AT); PAWLIK, Roland, 1130 Wien (AT); FUCHS, Florian, 2062 Seefeld-Kadolz (AT)
(74) Vertreter: Gramm, Lins & Partner Patent- und Rechtsanwälte PartGmbB
(86) Internationale Anmeldenummer: PCT/EP2016/072331
(87) Internationale Veröffentlichungsnummer: WO 2017/050779

(56) Entgegenhaltungen:
- DE-A1-102007 035 965
- US-A- 4 258 441
- US-A1- 2007 250 179

## Beschreibung

Die Erfindung betrifft eine Protheseneinrichtung mit einem an einer Kraftzugbandage befestigten Zugelement, das bei Aufbringen einer Zugkraft eine bewegliche Komponente der Protheseneinrichtung antreibt. Die Protheseneinrichtung ist insbesondere als Armprothese ausgebildet. Dokument US 4,258,441 A1 wird als nächstliegender Stand der Technik angesehen.

Prothesen ersetzen nicht vorhandene oder nicht mehr vorhandene Gliedmaßen. Neben rein kosmetischen Prothesen, die lediglich die Form der nichtvorhandenen Gliedmaße ersetzen, versuchen die überwiegende Anzahl moderner Prothesen eine oder mehrere Funktionen der Gliedmaße zu ersetzen oder eine ähnliche Funktionalität wie eine natürliche Gliedmaße bereitzustellen.

Prothesen für untere Extremitäten können als Prothesenfüße, die an einem Unterschenkelschaft befestigt sind, ausgebildet sein. Komplexere Prothesen ersetzen ein Kniegelenk, wobei es hiervon einachsigen Sperrkniegelenken über Mehrlenkerkniegelenke, computergesteuerte passive Kniegelenke und angetriebene Kniegelenke unterschiedliche Komplexitätsgrade gibt. Darüber hinaus gibt es Protheseneinrichtungen für Patienten mit Hüftexartikulation.

Die natürlichen oberen Extremitäten sind in der Lage, eine Vielzahl von Bewegungen auszuführen. Die Greif- und Haltefunktionen der Hand, verschiedene Rotationsbewegungen des Unterarms sowie die hohe Mobilität innerhalb des Schultergelenkes ermöglichen eine Vielzahl von Verrichtungen und Bewegungen, die nur schwer in einem künstlichen System abbildbar sind. Auch bei Protheseneinrichtungen der oberen Extremität existieren unterschiedliche Komplexitätsgrade, angefangen von einfachen Haken, über Greifer zu angetriebenen Prothesenhänden oder motorisch angetriebenen, myoelektrisch gesteuerten Armprothesen.

Neben hochkomplexen, computergesteuerten, sensorbasierten Protheseneinrichtungen existiert weiterhin ein Bedarf an vergleichsweise einfachen, mechanischen Protheseneinrichtungen, die über so genannte Kraftzugbandagen betätigt werden. Hierbei wird eine bewegliche Komponente einer Prothese der oberen Extremität durch eine Schulterbewegung, beispielsweise der Schulter der kontralateralen, unversorgten Seite, aktuiert. Dabei kann eine bewegliche Komponente in eine Richtung bewegt werden, entweder entgegen der Schwerkraft oder entgegen einer Gegenkraft, z.B. einer Federkraft. Werden aktive Funktionen einer Protheseneinrichtung allein über eine Kraft mittels einer Kraftzugbandage ausgelöst, nennt man solche Prothesen Eigenkraftprothesen. Im Vergleich zu Prothesen mit elektrischen Antrieben weisen Eigenkraftprothesen eine direkte Steuerbarkeit und ein propriozeptives Feedback auf.

Eine häufige Anwendung von Eigenkraftprothesen besteht darin, dass über die Kraftzugbandage ein Greifgerät oder Greifelement entgegen einer Gegenkraft, insbesondere Federkraft geöffnet wird. Die Gegenkraft kann über Druckfedern, Zugfedern oder Gummiringe oder pneumatisch oder auf andere Art und Weise aufgebracht werden und erzeugt somit die Griffkraft. Da bei vielen Tätigkeiten eine hohe Griffkraft von Vorteil ist, werden vergleichsweise starke Federn oder Gummiringe oder Kraft erzeugende Elemente montiert. Diese Gegenkraft muss beim Öffnen des Greifgerätes überwunden werden, also durch den Patienten mittels der Kraftzugbandage aufgebracht werden. Ein dauerhaftes Arbeiten entgegen der vergleichsweise hohen Gegenkräfte kann zu frühzeitiger Ermüdung oder gesundheitlichen Beschwerden des Patienten führen.

Die DE 26 39 143 C2 betrifft ein Getriebe für eine Orthese oder Prothese zur Umsetzung einer Rotationsbewegung eines Antriebsaggregates in eine Hin- und Herbewegung eines bewegten Teils einer Orthese oder Prothese. Das Getriebe weist ein drehbar gehaltenes, mit dem Antriebsaggregat gekoppeltes, ein Gewinde aufweisendes Bauteil und ein damit in Eingriff stehendes, unverdrehbar dem Gehäuse geführtes Getriebeteil auf. Das Bauteil besteht aus einem einseitig offenen Hohlzylinder mit einem Innengewinde und einem Außengewinde mit entgegengesetzten Steigungen. Das eine Ende eines Seilzuges oder Kettenzuges ist an einer mit dem Innengewinde zusammenwirkenden Schraubenspindel und das andere Ende an einer mit dem Außengewinde zusammenwirkenden Hülse festgelegt. Das Getriebe ist nach Art einer Doppelspindel ausgebildet, wodurch erreicht wird, dass über den gesamten Verschiebeweg die auf den Seil- oder Kettenzug wirkenden Zugkräfte im Wesentlichen konstant sind.

Die DE 821 690 B betrifft eine Prothesenhand mit einem hohlen Handkörper, in dem eine Kurvenscheibe angeordnet ist. Über Gestänge und Zuglaschen wird eine Drehung der Kurvenscheibe in eine Bewegung der Finger umgesetzt, um ein Öffnen oder Schließen der Hand zu bewirken.

Die DE 26 07 499 C3 betrifft eine Antriebsvorrichtung für die Finger einer künstlichen Hand, bei der ein beweglicher Daumen und mindestens ein dazu gegenläufig bewegter Finger mit Hilfe eines selbstsperrenden Getriebes motorisch angetrieben sind. Eine Schnecke befindet sich in direktem Eingriff mit an den beweglichen Fingern und dem Daumen befestigten Zahnrädern. Die Zahnräder kämmen mit der Schnecke an diametral gegenüberliegenden Seiten, wobei die Schnecke mit Hilfe eines Wechselgetriebes angetrieben ist.

Die US 4,604,098 A betrifft einen Prothesenarm mit einem Unterarmteil, einem Ellbogengelenk mit Verriegelungselementen und Eingriffselementen zum Verhindern einer Ellbogenbeugung und einem Oberarmteil, das über das Ellbogengelenk mit dem Unterarmteil verbunden ist. Über einen Motor wird der Unterarmteil relativ zu dem Oberarmteil um das Ellbogengelenk bewegt. Über ein Entriegelungsmechanismus können die Verriegelungsmittel außer Eingriff gebracht werden, um eine Beugung zu ermöglichen. Das Beugen sowie das Verriegeln oder Entriegeln wird elektronisch gesteuert.

Die DE 10 2007 035 965 A1 beschreibt eine rein motorisch angetriebene prothetische Greifeinheit, bei der über einen Bowdenzug ein Motor zum Antreiben der Greifeinheit aktuierbar ist. Durch Variation der auf den Bowdenzug ausgeübten Zugkraft ist es beispielsweise möglich, den Antrieb durch den Motor zu steuern und so die Greifkraft zu regulieren.

Aufgabe der vorliegenden Erfindung ist es daher, eine Protheseneinrichtung bereitzustellen, bei der ein ermüdungsfreies Arbeiten gewährleistet ist und eine geringe Belastung auf den Patienten ausgeübt wird.

Erfindungsgemäß wird diese Aufgabe durch eine Protheseneinrichtung mit den Merkmalen des Hauptanspruches gelöst, vorteilhafte Ausgestaltungen und Weiterbildungen der Erfindung sind in den Unteransprüchen, der Beschreibung und den Figuren offenbart.

Die erfindungsgemäße Protheseneinrichtung mit einem an einer Kraftzugbandage befestigten Zugelement, das bei Aufbringen einer Zugkraft eine bewegliche Komponente der Protheseneinrichtung antreibt, sieht vor, dass dem Zugelement eine Sensoreinrichtung zugeordnet ist, die die Betätigung des Zugelementes erfasst und einen der beweglichen Komponente zugeordneten Motor ansteuert. Über die Sensoreinrichtung wird erfasst, ob und wie Zugkräfte über die Kraftzugbandage auf das Zugelement aufgebracht werden. Die Sensoreinrichtung misst die Größe der aufgebrachten Zugkraft. Die Sensoreinrichtung leitet die entsprechend erzeugten Sensorsignale an eine Steuereinrichtung weiter, die mit der Sensoreinrichtung gekoppelt ist. Über diese Steuereinrichtung wird ein Motor angesteuert, also in Abhängigkeit von den Sensorsignalen in Betrieb gesetzt, um die bewegliche Komponente anzutreiben oder zumindest die beabsichtigte Bewegung der beweglichen Komponente zu unterstützen. Über die Kraftzugbandage erfolgt eine unmittelbare Rückkopplung an den Nutzer der Protheseneinrichtung, so dass die Vorteile einer herkömmlichen Eigenkraftprothese bestehen bleiben. Gleichzeitig wird die durch die Kraftzugbandage aufzubringende Kraft verringert, da zur Betätigung der beweglichen Komponente entgegen der Schwerkraft oder entgegen der Federkraft nur noch eine verringerte Kraft aufgebracht werden muss, da der Motor die intendierte Bewegung unterstützt. Anders als bei ausschließlich fremdkraftbetriebenen Prothesen ist es vorteilhaft, wenn über die Kraftzugbandage weiterhin eine Betätigungskraft auf die bewegliche Komponente ausgeübt wird. Ähnlich wie bei einer servounterstützten Lenkung wird die beabsichtigte Bewegung über das Zugelement ausgeführt, lediglich die von dem Patienten aufzubringende Kraft wird verringert.

Die bewegliche Komponente kann dabei bevorzugt als mechanisches Greif-element oder Greifgerät oder als gelenkige Komponente ausgebildet sein. Bevorzugt wird das mechanische Greifgerät entgegen einer Gegenkraft, insbesondere Federkraft geöffnet, bei Wegfallen der Zugkraft und der motorischen Unterstützung schließt das mechanische Greifgerät, so dass zu einem festen Greifen oder Fixieren eines Gegenstandes keine zusätzliche Kraft mehr aufgebracht werden muss. Die Haltekraft wird durch die Vorspannung oder das Kraft aufbringende Element ausgeübt. Grundsätzlich ist es auch möglich und vorgesehen, dass die Schließbewegung des Greifgerätes durch die Kraftzugbandage und das Zugelement ausgeführt wird, erfindungsgemäß mit motorischer Unterstützung. Bei Wegfall der Zugkraft und der motorischen Unterstützung wird das Greifgerät dann infolge einer Federkraft geöffnet, bei einer entgegengesetzten Ausgestaltung entgegen einer Federkraft geöffnet. Alternativ oder ergänzend zu einem rein mechanischen Greifgerät ist es vorgesehen, dass auch eine Gelenkkomponente der Protheseneinrichtung auf eine solche Art angetrieben wird. Eine Verlagerung der Prothesenkomponente in eine bestimmte Richtung, sei es gegen eine Federkraft oder gegen die Schwerkraft, ist vorgesehen. Beispielsweise können Prothesenkomponenten um eine Schwenkachse entgegen Federkräfte verlagert werden, wobei die Federkräfte die Prothesenkomponente in einer Ausgangslage halten. Je nach Kraftrichtung kann dann in die eine oder andere Richtung eine Verschwenkung oder Verdrehung der Prothesenkomponente stattfinden. Das mechanische Greifgerät kann zwei oder mehr hakenförmige Greifelemente aufweisen und insbesondere als sogenannter Hook ausgebildet sein. Neben einem Zweifingergreifer ist es auch möglich, eine Prothesenhand als Greifgerät vorzusehen.

Eine Weiterbildung der Erfindung sieht vor, dass die aufgebrachte Unterstützungskraft durch den Motor proportional zu der durch das Zugelement aufgebrachten Zugkraft ist. Dadurch wird die direkte propriozeptive Rückkopplung gewährleistet, je mehr Kraft über die Kraftzugbandage aufgebracht wird, desto größer wird einerseits die auf die bildliche Komponente aufgebrachte Zugkraft und andererseits die über den Motor zusätzlich aufgebrachte Unterstützungskraft erhöht. Insbesondere bei einem Schließen des mechanischen Greifgerätes durch die Zugkraft und die Motorunterstützung ergeben sich die geschilderten Vorteile der direkten Rückkopplung. Ebenso sind der Druckpunkt und der Griffpunkt einstellbar.

Bevorzugt ist der Motor über ein Getriebe mit dem Zugelement und/oder der beweglichen Komponente gekoppelt. Die Zwischenschaltung von einem oder mehreren Getrieben ermöglicht es, kleine, schnelllaufende und leichte Motoren einzusetzen, was im Hinblick auf den bevorzugten Einsatzzweck bei Prothesen der oberen Extremität besonders günstig ist, da dort wenig Raum zur Montage von Motoren und Energiespeichern vorhanden ist. Als Getriebe können Seilwickelgetriebe, Zahnriemengetriebe, Zahnradgetriebe, Trommelgetriebe oder Reibradgetriebe oder Planetengetriebe eingesetzt werden. Insbesondere Seilwickelgetriebe sind vorteilhaft einzusetzen, da bei herkömmlichen mechanischen Greifgeräten die Kraftübertragung von der kontralateralen Schulter zu dem mechanischen Greifgerät über einen Seilzug, gegebenenfalls mit Umlenkrollen stattfindet.

Sowohl die Getriebeübersetzung als auch der Proportionalitätsfaktor sind vorteilhafterweise einstellbar, um eine Anpassung an unterschiedliche Einsatzzwecke zu erreichen. Ebenso ist es vorteilhaft, wenn der Proportionalitätsfaktor der Unterstützungskraft durch den Motor einstellbar ist, ebenso wie die Kraftverstärkung oder die Unterstützungsleistung durch den Motor einstellbar ist. So kann bei einer nicht benötigten Unterstützung der Motor mit weniger Energie versorgt oder abgekoppelt werden, wodurch sich die Einsatzdauer der Protheseneinrichtung verlängert, da nur so viel Unterstützungsenergie bereitgestellt wird, wie benötigt wird.

Eine Weiterbildung der Erfindung sieht vor, dass die bewegliche Komponente mit einer Federkraft belastet ist, die dem Zugelement und/oder dem Motor entgegenwirkt. Dadurch ist es möglich, dass Öffnen oder Schließen der Greifeinrichtung bzw. das Beugen oder Strecken der Gelenkkomponente zu beeinflussen und insbesondere bei einer Greifeinrichtung eine definierte Haltekraft ohne zusätzliche Bewegung oder einen zusätzlichen Energieeintrag zu ermöglichen. Bei Gelenkeinrichtungen kann dadurch eine Stellung der Komponenten zueinander als Vorzugsstellung eingerichtet werden, die erst durch Aufbringen einer Kraft ab einem festlegbaren Schwellwert veränderbar ist.

Die Sensoreinrichtung kann einen Kraftsensor in dem Zugelement oder in einer Umlenkrolle aufweist, um eine angepasste Zusatzkraft bereitstellen zu können, die beispielsweise proportional zu der durch den Nutzer aufgebrachten Kraft ist. Der Proportionalitätsfaktor muss über die Kraft nicht linear sein, die zusätzliche Kraft über den Motor kann bei steigender Kraft überproportional angehoben werden. Auch ist es möglich, dass die maximal aufgebrachte Kraft begrenzt wird, also dass die Summe der über das Zugelement oder der auf die bewegliche Komponente übertragenen Kraft auf einen Maximalwert begrenzt wird.

In einer Ausgestaltung der Erfindung ist die Sensoreinrichtung oder der Kraftsensor als Kraftmessbolzen ausgebildet ist. Der Sensor ist bevorzugt als Kraftmessbolzen innerhalb des Zugelementes ausgebildet, so dass eine einfache Ermittlung der innerhalb des Zugelementes wirkenden Kraft gegeben ist. Über einen Signalverstärker kann die Kraft gemessen und die zusätzlich zuzuführende Kraft durch den Motor proportional zur Zugkraft aufgebracht werden. Neben einer linearen Proportionalität können auch progressive oder degressive Proportionalitäten vorgesehen sein, um beispielsweise bei besonders hohen aufgebrachten Kräften durch die Zugkraft eine besonders große Unterstützung zu bewirken.

Alternativ oder ergänzend zu einer direkten Anordnung eines Sensors innerhalb des Zugelementes kann eine Kraftmesseinrichtung auch in einer Umlenkrolle oder an einem Getriebeelement angeordnet sein, um dort die über das Zugelement beziehungsweise die Kraftzugbandage aufgebrachte Zugkraft zu erfassen und den Motor anzusteuern.

Der Motor kann über eine Kupplung dem Zugelement und/oder der beweglichen Komponente zugeordnet sein, so dass bei einem Ausfall des Motors oder bei Erschöpfung der Energiespeicher die bewegliche Komponente weiterhin wie gewohnt von dem Zugelement betätigt werden kann. Der Motor ist somit zwischen das Zugelement und der beweglichen Komponente zuschaltbar oder gegebenenfalls davon entkoppelbar. Die Kupplung kann beispielsweise über eine Fliehkraftkupplung oder eine federbelastete Ausrückkopplung realisiert werden.

Über eine Kupplung ist es auch möglich, die Protheseneinrichtung modular aufzubauen, das heißt, eine herkömmliche Eigenkraftprothese mit einer Zusatzkomponente in Gestalt des Motors mit der Steuerungseinrichtung als Zubehörkomponente anzubieten, um eine zusätzliche und verbesserte Funktionalität der Protheseneinrichtung zu ermöglichen.

Die bewegliche Komponente, insbesondere das mechanische Greifgerät, ist auswechselbar an der Protheseneinrichtung befestigt, um unterschiedliche Funktionalitäten für unterschiedliche Verrichtungen bereitstellen zu können.

Das Zugelement kann als Seilzug oder Gurt ausgebildet sein, das über die Kraftzugbandage mit betätigt wird. Das Zugseil oder Zugelement kann in einer Seilhülle oder Hülle geführt sein, wobei das Endstück frei und über eine Umlenkrolle und gegebenenfalls Unterstützungsrolle an der beweglichen Komponente, insbesondere der mechanischen Greifeinrichtung festgelegt ist.

Das Zugelement kann zumindest teilweise innerhalb eines Prothesenschaftes geführt sein, wobei die Anpassung des Zugelementes über einen Orthopädie-techniker an den jeweiligen Patienten oder Nutzer erfolgt. Sowohl die Länge des Zugelementes als auch die Übersetzungen und Verstärkungen werden individuell an den jeweiligen Patienten angepasst und sind einstellbar.

Der Motor kann auf das Zugelement einwirken und somit die über das Zugelement auf die bewegliche Komponente übertragene Zugkraft erhöhen, also das Zugelement als einziges Kraftüberragungselement auf die bewegliche Komponente nutzen. Alternativ kann der Motor separat von dem Zugelement mit der beweglichen Komponente gekoppelt sein und beispielsweise über ein Zahnradgetriebe oder ein Reibradgetriebe, ggf. mit einem dazwischengeschalteten Freilauf direkt mit der beweglichen Komponente gekoppelt sein. Die Kraftübertragung von dem Motor auf die bewegliche Komponente erfolgt dann nicht über das Zugelement, sondern gesondert, wodurch keine Änderungen in dem Aufbau der ursprünglichen Protheseneinrichtung notwendig werden. Lediglich die Sensoreinrichtung muss dem Zugelement zugeordnet und die Unterstützungseinrichtung mit Motor, ggf. Getriebe und Steuerung mit der beweglichen Komponente mechanisch kraftübertragend gekoppelt werden.

Nachfolgend werden Ausführungsbeispiele der Erfindung genannt, der die beigefügten Figuren näher erläutert. Es zeigen:
- Figur 1: - eine bewegliche Komponente in Gestalt eines Greifgerätes; sowie
- Figur 2: - eine schematische Darstellung der Protheseneinrichtung.

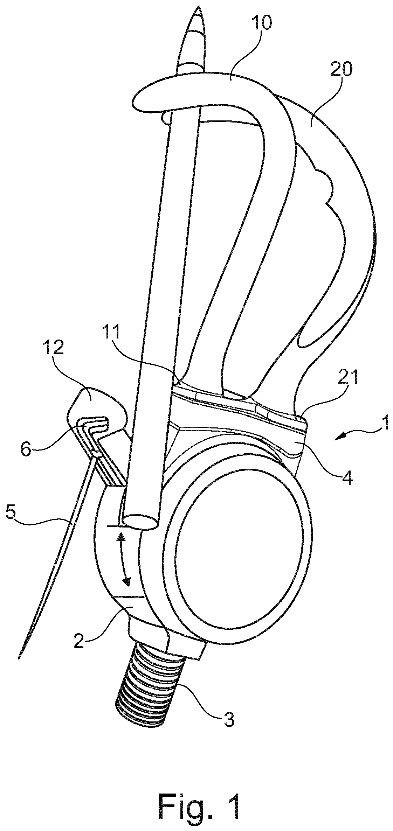

In der Figur 1 ist in einer Einzeldarstellung eine bewegliche Komponente 1 in Gestalt eines mechanischen Greifgerätes dargestellt, das als sogenannter Hook ausgebildet ist. Das mechanische Greifgerät 1 weist einen Grundkörper 2 auf, an dessen einem Ende eine Befestigungseinrichtung 3 in Gestalt eines Gewindes zur Festlegung an einem nicht dargestellten Prothesenschaft angeordnet oder ausgebildet ist. An dem der Befestigungseinrichtung 3 gegenüberliegenden Ende des Grundkörpers 2 sind zwei Ausleger 10, 20 befestigt, die hakenförmig und zum Greifen von Gegenständen, in der Figur 1 in Gestalt eines Stiftes, ausgebildet sind. Ein erster Ausleger 20 ist starr an dem Grundkörper 2 befestigt, der zweite Ausleger 10 ist verschwenkbar an dem Grundkörper 2 gelagert, wobei die nicht eingezeichnete Schwenkachse dergestalt ausgebildet ist, dass der zweite Ausleger 10 von dem ersten Ausleger 20 wegbewegt werden kann, so dass ein Zwischenraum zwischen den beiden Auslegern 10, 20 vergrößert beziehungsweise verkleinert werden kann. Die Verlagerung des zweiten Auslegers 10 von dem ersten Ausleger 20 weg erfolgt entgegen einer Gegenkraft, die die beiden Ausleger 10, 20 im nicht betätigten Zustand aneinander anliegen lässt. Die Gegenkraft wird über ein Gummiring 4 ausgeübt, der um eine jeweilige Basis 11, 21 für den zweiten Ausleger 10 beziehungsweise ersten Ausleger 20 herumgelegt ist. Der Gummiring 4 ist in Nuten eingelegt und ist somit auswechselbar an den beiden Basen 11, 21 gelagert. Je nach Einsatzzweck und benötigter Haltekraft, die zwischen den beiden Auslegern 10, 20 ausgeübt werden soll, können unterschiedliche Gummiringe 4 in die Nut eingelegt werden. Wird nur eine geringe Haltekraft benötigt, wird ein nachgiebiger Gummiring 4 eingelegt, wird eine hohe Haltekraft benötigt, wird ein stabilerer Gummiring 4 mit einer geringeren Dehnbarkeit und Elastizität und somit über einem höheren Widerstand gegen eine Auslenkung verwendet.

Die Ausleger 10, 20 können auswechselbar an der jeweiligen Basis 11, 21 befestigt sein, beispielsweise eingeschraubt oder auf andere Art und Weise eingesetzt und formschlüssig fixiert.

Zur Bewegung des zweiten Auslegers 10 von dem ersten Ausleger 20 weg ist ein Zugelement 5 an einem dritten Ausleger 12 befestigt, der starr mit dem zweiten Ausleger 10 gekoppelt ist. Das Zugelement 5 ist in Gestalt eines Zuggurtes oder Kabels oder Seilzug ausgebildet und formschlüssig an dem dritten Ausleger 12 in einer darin ausgebildeten Nut 6 gelagert. Die Nut 6 erstreckt sich entlang der proximalen Seite des dritten Auslegers 12, also derjenigen Seite, die dem Prothesenschaft zugewandt ist, und ermöglicht eine Veränderung des Hebelverhältnisses bei der Kraftübertragung von dem Zugelement 5 auf den zweiten Ausleger 10. Je weiter das Zugelement 5 nach außen verlagert wird, desto größer ist der Hebelweg, wodurch eine Verringerung der aufzubringenden Kraft bei gleichzeitiger Verringerung des Öffnungsweges zwischen den Auslegern 10, 20 erreicht wird. Der dritte Ausleger 12 ist mit der zweiten Basis 11 gekoppelt und steht von dieser ab, so dass durch Aufbringung einer Zugkraft durch das Zugelement 5 die zweite Basis 11 und damit auch der zweite Ausleger 10 um die Schwenkachse verschwenkt wird. Die Schwenkachse ist im Wesentlichen senkrecht zu den im Wesentlichen kreisförmigen Hauptflächen des scheibenartigen Grundkörpers 2 ausgerichtet. Wird an dem Zugelement 5 gezogen, bewegt sich der dritte Ausleger 12 und damit auch der zweite Ausleger 10 in Pfeilrichtung nach unten, wird die Zugkraft verringert, bewegt sich der dritte Ausleger 12 und damit auch der zweite Ausleger 10 in Pfeilrichtung nach oben, da der Gummiring 4 die entsprechende Gegenkraft ausübt. Der zu greifende Gegenstand, im dargestellten Ausführungsbeispiel ein Stift, wird zwischen dem ersten Ausleger 20 und dem zweiten Ausleger 10 gehalten und kann auf dem dritten Ausleger 12 abgelegt werden.

Die Betätigung des Zugelementes 5 kann bei einer Eigenkraftprothese ausschließlich über eine Verlagerung eines Körperteils erfolgen, beispielsweise der Schulter des versorgten oder nicht versorgten Armes.

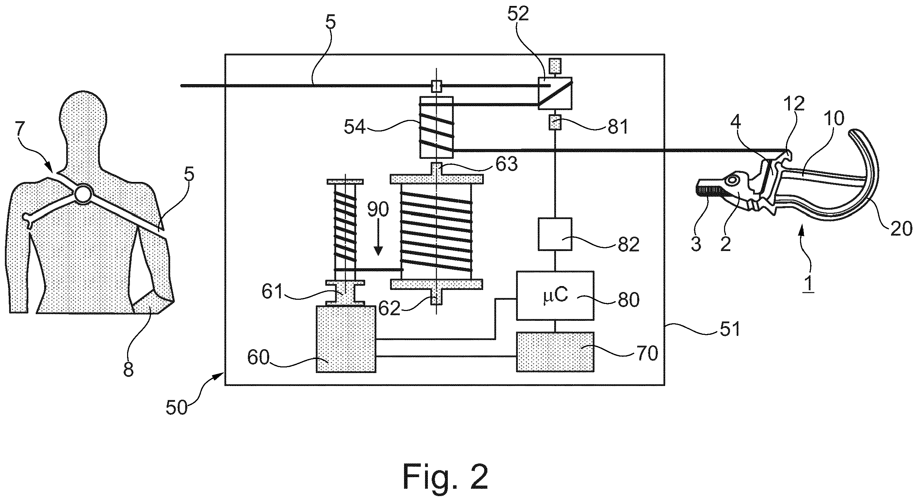

In der Figur 2 ist die Protheseneinrichtung in einem schematischen Aufbau gezeigt. An einem Patienten, der links in der schematischen Darstellung aufgeführt ist, ist eine Kraftzugbandage 7 angelegt, die mit einem Prothesenschaft 8 gekoppelt ist. Die Kraftzugbandage 7 ist auf der kontralateralen, unversorgten Schulter festgelegt und schlaufenartig um die Schulter herumgeführt. Am Rücken, im Bereich der oberen Brustwirbel, ist die Schlaufe in einem Ring befestigt, an dem ein Zugelement 5 in Gestalt eines Zuggurtes befestigt ist. Wird die unversorgte Schulter nach vorne geführt, bewirkt dies, dass auf das Zugelement 5 eine Zugkraft ausgeübt wird. Das Zugelement 5 ist an dem Oberarm auf der versorgten Seite so entlanggeführt, dass das Zugelement 5 innerhalb des Prothesenschaftes 8 entlang geführt wird. In der dargestellten Ausführungsform ist das Zugelement 5 zunächst als ein Gurt ausgebildet, der dann innerhalb des Prothesenschaftes 8 mit einem seilförmigen Zugelement 5, beispielsweise einem Drahtseil, einer Litze, der Seele eines Bowdenzuges oder dergleichen gekoppelt ist oder in ein Zugelement 5 solcher Art übergeht.

Innerhalb des Prothesenschaftes 8 ist eine Unterstützungseinrichtung 50 angeordnet, die schematisch und vergrößert dargestellt ist. Die Unterstützungseinrichtung 50 kann als Modul aufgebaut sein und ein Gehäuse 51 aufweisen, innerhalb dessen das von der Kraftzugbandage 7 kommende seilförmige Zugelement 5 geführt ist. Das Zugelement 5 ist um eine Umlenkrolle 52 herumgeführt, die mit einer Sensoreinrichtung 81 in Gestalt eines Kraftmessbolzens gekoppelt ist. Von der Umlenkrolle 52 wird das Zugelement 5 über eine Unterstützungsrolle 54 geführt und von dort an dem dritten Ausleger 12 der beweglichen Komponente 1 befestigt. In der Figur 2 ist eine alternative Ausgestaltung der beweglichen Komponente in Gestalt eines mechanischen Greifgerätes 1 dargestellt, bei der der Grundkörper 2 nicht kreisscheibenförmig und das die Gegenkraft erzeugende Element 4 nicht als Gummiring, sondern mit einer dreieckigen Kontur und als Zugfeder ausgebildet sind. Wird das Zugelement 5 von der Kraftzugbandage 7 mit einer Zugkraft in Richtung von dem mechanischen Greifgerät 1 weg beaufschlagt, wird die Umlenkrolle 52 gedreht, ebenso die Unterstützungsrolle 54 und darüber eine Verlagerung der beiden Ausleger 10, 20 voneinander weg bewirkt.

Innerhalb der Unterstützungseinrichtung 50 ist ein Elektromotor 60 mit einer Antriebswelle 61 angeordnet. Der Motor 60 wird über einen elektrischen Energiespeicher 70 mit elektrischer Energie versorgt. Dem Motor 60 ist eine Steuereinrichtung 80 zugeordnet, über die der Motor 60 aktiviert oder deaktiviert wird. In dem dargestellten Ausführungsbeispiel ist der Umlenkrolle 52 eine Sensoreinrichtung 81 in Gestalt eines Kraftmessbolzens zugeordnet. Wird eine Zugkraft auf das Zugelement 5 ausgeübt, wird die Umlenkrolle 52 mit einem Drehmoment und einer Kraft beaufschlagt, die über die Sensoreinrichtung 81 gemessen wird. Über ein Signalverstärker 82 wird das Signal zu der Steuereinrichtung 80 geleitet. Wird eine Zugkraft innerhalb des Zugelementes 5 an der Umlenkrolle 52 detektiert, wird der Motor 60 über die Steuereinrichtung 80 aktiviert. Die Antriebswelle 61 wird angetrieben. Mit der Antriebswelle 61 ist eine Abtriebswelle 62 über ein Seilwickelgetriebe 90 gekoppelt. Die Antriebswelle 61 wickelt ein Kupplungsseil auf und dreht darüber die Abtriebswelle 62, die über eine Kupplung 63 mit der Unterstützungsrolle 54 gekoppelt ist. Die Kupplung 63 kann als Fliehkraftkupplung oder federbelastete Zahnradkupplung ausgebildet sein. Ebenso ist es möglich, dass die Kupplung eine sägezahnartige Stirnverzahnung aufweist, die so orientiert ist, dass eine Kraftübertragung nur in Richtung der Unterstützung der Zugkraft des Zugelementes 5 erfolgt.

Im dargestellten Ausführungsbeispiel muss die Unterstützungsrolle 54 zum Öffnen des mechanischen Greifgeräts 1 nach links drehen, die Flanken der sägezahnartigen Stirnverzahnung würden dann nach rechts flach abfallen. Statt eines dargestellten Seilwickelgetriebes können Zahnradgetriebe, Zahnriemengetriebe, Trommelgetriebe oder Reibradgetriebe oder Planentengetriebe zwischen dem Motor 60 und der Unterstützungsrolle 54 ausgebildet sein. Über die unterschiedlichen Durchmesser der Antriebswelle 61 und der Abtriebswelle 62 können unterschiedliche Übersetzungen des Seilwickelgetriebes 90 realisiert werden, eine Anpassung kann leicht durch Auswechseln der jeweiligen Wellen oder der Trommeln auf den jeweiligen Wellen 61, 62 und/oder der Unterstützungsrolle 54 erfolgen.

Die durch den Motor 60 aufgebrachte Unterstützungskraft kann über die Steuereinheit 80 eingestellt werden. Die Einstellung kann in Abhängigkeit von dem geplanten Einsatzzweck des mechanischen Greifgerätes 1 oder der persönlichen Vorlieben des Nutzers erfolgen.

Statt eines Kraftmessbolzens 81 an der Umlenkrolle 52 ist es auch möglich und vorgesehen, dass die Sensoreinrichtung 8 im Verlauf des Zugseils 5 angeordnet ist, beispielsweise in Gestalt einer Zugkraftsensoreinrichtung, die die im Zugelement 5 wirksame Zugkraft ermittelt. Die Übermittlung der Sensordaten kann drahtlos an den Signalverstärker 82 oder auch drahtgebunden erfolgen. Das Zugelement 5 kann über dem größten Teil einer Länge innerhalb einer Umhüllung geführt sein, um ein Scheuern des Zugelementes 5 auf der Haut oder auf der Kleidung des Nutzers zu vermeiden.

Die Unterstützungseinrichtung 50 kann als Modul ausgebildet sein und einfach an eine bestehende Eigenkraftprothese angebaut werden. Das Zugelement 5 muss zum Einbau lediglich um die Umlenkrolle 2 sowie die Unterstützungsrolle 54 gelegt werden, was nur eine geringe Verlängerung des Zugelementes 5 gegenüber einer direkten Befestigung an dem mechanischen Greifgerät 1 bedeutet. Sollte der Energiespeicher 70 leer sein oder der Motor 4 einen Defekt aufweisen, kann die Protheseneinrichtung weiterhin genutzt werden, ohne dass es zu einer grundsätzlichen Funktionseinschränkung kommt, lediglich der Komfort wird verringert.

Statt eine Kraftaufbringung durch den Motor 4 über das Zugelement 5 zu bewirken, ist in einer alternativen Ausgestaltung der Antrieb unmittelbar mit dem beweglichen zweiten Ausleger 10 gekoppelt. Die Unterstützungseinrichtung 50 kann in dem Grundkörper 2 integriert werden und über eine Getriebeanordnung, beispielsweise ein Reibradgetriebe oder ein Zahnradgetriebe mit entsprechender Kupplung 63 kraftübertragend mit dem beweglichen Ausleger 10 gekoppelt sein. Wird dann über die Sensoreinrichtung 81, die auch direkt in dem Zugelement 5 angeordnet sein kann, eine Zugkraft detektiert, kann die Sensoreinrichtung 81 dies per Kabel oder drahtlos an den Signalverstärker 82 oder die Steuerung 80 übermitteln, woraufhin dann der Motor 60 aktiviert wird. Auf diese Weise ist es möglich, auf eine Beanspruchung des Zugelementes 5 durch die zusätzlich aufgebrachte Motorkraft zu verzichten. Änderungen in der Anlenkung des Zugelementes 5 an dem mechanischen Greifgerät 1 werden nicht notwendig. Die Unterstützungseinrichtung 50 wird als gesonderte, mechanische Komponente mit dem Motor 60 und der integrierten Steuerung 80 zusammen mit dem Energiespeicher 70 als vorgefertigtes Modul bereitgestellt und muss lediglich in dem Grundkörper 2 oder an dem Grundkörper 2 befestigt werden. Über eine geeignete Kupplung 63 wird dann die Kraft des Motors 60 zum Antreiben des beweglichen Auslegers 10 übertragen. Die Größe der aufzubringenden Kraft ebenso wie die Dauer werden über die Zugkräfte bestimmt, die in der Sensoreinrichtung 81 ermittelt werden. Es kann eine lineare Proportionalität zwischen der aufgebrachten Zugkraft über das Zugelement 5 und der zusätzlich bereitgestellten Motorkraft eingestellt werden, alternative Proportionalitätsfaktoren sind möglich. Den beiden Ausführungsformen ist gemeinsam, dass bei einem Ausfall der Unterstützungseinrichtung 50 die Funktionalität des mechanischen Greifgeräts 1 aufrechterhalten bleibt.

## Patentansprüche

1. Protheseneinrichtung mit einem an einer Kraftzugbandage (7) befestigten Zugelement (5), das beim Aufbringen einer Zugkraft eine bewegliche Komponente der Protheseneinrichtung antreibt, **dadurch gekennzeichnet, dass** dem Zugelement (5) eine Sensoreinrichtung (81) zugeordnet ist, die die Betätigung des Zugelementes (5) erfasst und einen der beweglichen Komponente (1) zugeordneten Motor (60) ansteuert.

2. Protheseneinrichtung nach Anspruch 1, **dadurch gekennzeichnet, dass** die bewegliche Komponente (1) als mechanisches Greifgerät oder Gelenkkomponente ausgebildet ist.

3. Protheseneinrichtung nach Anspruch 1 oder 2, **dadurch gekennzeichnet, dass** die aufgebrachte Unterstützungskraft durch den Motor (60) proportional zu der durch das Zugelement (5) aufgebrachten Zugkraft ist.

4. Protheseneinrichtung nach einem der voranstehenden Ansprüche, **dadurch gekennzeichnet, dass** der Motor (60) über ein Getriebe (90) mit dem Zugelement (5) und/oder der beweglichen Komponente (1) gekoppelt ist.

5. Protheseneinrichtung nach Anspruch 4, **dadurch gekennzeichnet, dass** das Getriebe (90) als Seilwickelgetriebe, Zahnriemengetriebe, Zahnradgetriebe, Trommelgetriebe, Reibradgetriebe oder Planetengetriebe ausgebildet ist.

6. Protheseneinrichtung nach Anspruch 4 oder 5, **dadurch gekennzeichnet, dass** die Getriebeübersetzung und der Proportionalitätsfaktor einstellbar sind.

7. Protheseneinrichtung nach einem der voranstehenden Ansprüche, **dadurch gekennzeichnet, dass** die Unterstützungskraft durch den Motor (60) einstellbar ist.

8. Protheseneinrichtung nach einem der voranstehenden Ansprüche, **dadurch gekennzeichnet, dass** die bewegliche Komponente (1) mit einer Federkraft belastet ist, die dem Zugelement (5) und/oder dem Motor (60) entgegenwirkt.

9. Protheseneinrichtung nach einem der voranstehenden Ansprüche, **dadurch gekennzeichnet, dass** die Sensoreinrichtung (81) einen Kraftsensor in dem Zugelement (5) oder in einer Umlenkrolle (52) aufweist.

10. Protheseneinrichtung nach einem der voranstehenden Ansprüche, **dadurch gekennzeichnet, dass** der Kraftsensor (81) als Kraftmessbolzen ausgebildet ist.

11. Protheseneinrichtung nach einem der voranstehenden Ansprüche, **dadurch gekennzeichnet, dass** der Motor (60) über eine Kupplung (63) dem Zugelement (5) und/oder der beweglichen Komponente (1) zugeordnet ist.

12. Protheseneinrichtung nach einem der voranstehenden Ansprüche, **dadurch gekennzeichnet, dass** der Motor (60) das Zugelement (5) antreibt oder die bewegliche Komponente (1) gesondert antreibt.

## Claims

1. A prosthesis device with a tensioning element (5) which is fastened to a body harness (7) and which drives a movable component of the prosthesis device when a tensile force is applied, **characterized in that** the tensioning element (5) is assigned a sensor device (81) which detects the actuation of the tensioning element (5) and activates a motor (60) assigned to the movable component (1).

2. The prosthesis device as claimed in claim 1, **characterized in that** the movable component (1) is configured as a mechanical gripper or joint component.

3. The prosthesis device as claimed in claim 1 or 2, **characterized in that** the support force applied by the motor (60) is proportional to the tensile force applied by the tensioning element (5).

4. The prosthesis device as claimed in one of the preceding claims, **characterized in that** the motor (60) is coupled to the tensioning element (5) and/or to the movable component (1) via a gear (90).

5. The prosthesis device as claimed in claim 4, **characterized in that** the gear (90) is configured as a cable winding gear, toothed belt gear, toothed wheel gear, drum gear, friction gear or planetary gear.

6. The prosthesis device as claimed in claim 4 or 5, **characterized in that** the geared transmission and the proportionality factor are adjustable.

7. The prosthesis device as claimed in one of the preceding claims, **characterized in that** the support force provided by the motor (60) is adjustable.

8. The prosthesis device as claimed in one of the preceding claims, **characterized in that** the movable component (1) is loaded with a spring force which counteracts the tensioning element (5) and/or the motor (60).

9. The prosthesis device as claimed in one of the preceding claims, **characterized in that** the sensor device (81) has a force sensor in the tensioning element (5) or in a deflection roller (52).

10. The prosthesis device as claimed in one of the preceding claims, **characterized in that** the force sensor (81) is configured as a force-measuring bolt.

11. The prosthesis device as claimed in one of the preceding claims, **characterized in that** the motor (60) is assigned to the tensioning element (5) and/or to the movable component (1) via a coupling (63).

12. The prosthesis device as claimed in one of the preceding claims, **characterized in that** the motor (60) drives the tensioning element (5) or separately drives the movable component (1).

## Revendications

1. Ensemble prothétique comportant un élément de traction (5) fixé à un bandage à force de traction (7) et entraînant un composant mobile de l'ensemble prothétique lors de l'application d'une force de traction,
**caractérisé en ce que**
un moyen capteur (81) est associé à l'élément de traction (5), qui détecte l'actionnement de l'élément de traction (5) et qui pilote un moteur (60) associé au composant mobile (1).

2. Ensemble prothétique selon la revendication 1,
**caractérisé en ce que**
le composant mobile (1) est réalisé sous la forme d'un appareil de saisie mécanique ou d'un composant articulé.

3. Ensemble prothétique selon la revendication 1 ou 2,
**caractérisé en ce que**
la force d'assistance appliquée par le moteur (60) est proportionnelle à la force de traction appliquée par l'élément de traction (5).

4. Ensemble prothétique selon l'une des revendications précédentes,
**caractérisé en ce que**
le moteur (60) est couplé à l'élément de traction (5) et/ou au composant mobile (1) par un mécanisme (90).

5. Ensemble prothétique selon la revendication 4,
**caractérisé en ce que**
le mécanisme (90) est réalisé sous forme de mécanisme à câble enroulé, de mécanisme à courroie crantée, de mécanisme à roue dentée, de mécanisme à tambour, de mécanisme à roue de friction ou de mécanisme planétaire.

6. Ensemble prothétique selon la revendication 4 ou 5,
**caractérisé en ce que**
le rapport de démultiplication et le facteur de proportionnalité sont réglables.

7. Ensemble prothétique selon l'une des revendications précédentes,
**caractérisé en ce que**
la force d'assistance par le moteur (60) est réglable.

8. Ensemble prothétique selon l'une des revendications précédentes,
**caractérisé en ce que**
le composant mobile (1) est chargé par une force élastique qui s'oppose à l'élément de traction (5) et/ou au moteur (60).

9. Ensemble prothétique selon l'une des revendications précédentes,
**caractérisé en ce que**
le moyen capteur (81) comprend un capteur de force dans l'élément de traction (5) ou dans un galet de renvoi (52).

10. Ensemble prothétique selon l'une des revendications précédentes,
**caractérisé en ce que**
le capteur de force (81) est réalisé sous forme de boulon de mesure de force.

11. Ensemble prothétique selon l'une des revendications précédentes,
**caractérisé en ce que**
le moteur (60) est associé à l'élément de traction (5) et/ou au composant mobile (1) par un accouplement (63).

12. Ensemble prothétique selon l'une des revendications précédentes,
**caractérisé en ce que**
le moteur (60) entraîne séparément l'élément de traction (5) ou le composant mobile (1).
